# EUROPEAN PATENT SPECIFICATION

(11) **EP 2 844 766 B1**
(45) Date of publication and mention of the grant of the patent: **23.11.2016**
(21) Application number: 13724179.0
(22) Date of filing: 29.04.2013
(51) Int. Cl.: C12Q 1/68

(54) **TARGETED DNA ENRICHMENT AND SEQUENCING**
GEZIELTE DNA-ANREICHERUNG UND -SEQUENZIERUNG
ENRICHISSEMENT ET SÉQUENÇAGE D'ADN CIBLÉ

(30) Priority: 30.04.2012 EP 12166190
(43) Date of publication of application: 11.03.2015
(73) Proprietor: Qiagen GmbH, 40724 Hilden (DE)
(72) Inventor: WEDLER, Holger, 40724 Hilden (DE); WEDLER, Erika, 40724 Hilden (DE); LÖFFERT, Dirk, 40589 Düsseldorf (DE); O'NEIL, Dominic, 40724 Hilden (DE)
(74) Representative: CH Kilger Anwaltspartnerschaft mbB
(86) International application number: PCT/EP2013/058934
(87) International publication number: WO 2013/164319

(56) References cited:
- WO-A1-2011/097528
- WO-A2-2007/056723

## Description

### FIELD OF THE INVENTION

The present invention is in the field of molecular biology, nucleic acid sequencing and more in particular DNA sequence enrichment and sequencing.

### BACKGROUND

Over the years, research in the field of genome analysis has progressed from sequencing only a few nucleotides to sequencing whole genomes.

High-throughput sequencers, also called 'next-generation' ('next-gen' or 'ngs'), or sometimes 'second-generation' (as opposed to third generation) sequencers are technologies that deliver 10⁵ to several 10⁶ of DNA reads, covering millions of bases or Gbp. It is being used to (re)sequence genomes, determine the DNA-binding sites of proteins (ChIP-seq), sequence transcriptomes (RNA-seq) (see last paragraph).

Manufacturers and technologies are Solexa/Illumina which generate up to 600 Gigabase (Gb) reads of 36 or 150 bp, Roche/454 which generate up to 700 Mbp reads of 400 - 1000 bp, ABI/SOLiD which generate > 20 Gb/day reads of 35-75 bp, Helicos which generate 21-35 Gb reads of 25-45 bp and Complete Genomics (a service company).

These technologies bring analysis of sequence information to another level. Rethinking experiments is crucial.

For example, if one wanted to analyse all known oncogenes (approximately 3000 genes related to cancer are known [M.E.Higgins et al. CancerGenes: a gene selection resource for cancer genome projects. Nature Methods.2007 35(1). Pp. D721-D726]) one would have to sequence a huge amount of DNA for a small amount of relevant sequence information.

The great amount of data generated makes it crucial to plan experiments in such a way that primarily useful sequence information is generated.

It is therefore an object of the present invention to provide a method for enriching only those DNA sequences which are of interest (target sequences). It is further an object of the present invention to provide a method for specifically determining the sequences of the target sequences without the need to sequence all DNA present in a (complex) sample.

### DEFINITIONS

A "composition" herein is an aqueous solution comprising at least one or more deoxyribonucleic acid molecules (DNA molecules). Preferably, the composition is a complex solution, i.e. a solution comprising DNA sequences of interest (target sequences) and further DNA sequences which are not of interest (unwanted sequences). As will be obvious to the skilled person, the unwanted sequences are usually much more abundant than the target sequences differing by 1, 2, 3, 4, 5, 6, 7, 8, 9, 10, 11, 12, 13, 14, 15 or more orders of magnitudes.

A "ribonucleic acid" herein contains in each nucleotide a ribose sugar, with carbons numbered 1' through 5'. A base is attached to the 1' position, in general, adenine (A), cytosine (C), guanine (G), or uracil (U). Adenine and guanine are purines, cytosine, and uracil are pyrimidines. A phosphate group is attached to the 3' position of one ribose and the 5' position of the next. The phosphate groups have a negative charge each at physiological pH, making RNA a charged molecule (polyanion). The bases may form hydrogen bonds between cytosine and guanine, between adenine and uracil and between guanine and uracil. However, other interactions are possible, such as a group of adenine bases binding to each other in a bulge, or the GNRA tetraloop that has a guanine-adenine base-pair. An important structural feature of RNA that distinguishes it from DNA is the presence of a hydroxyl group at the 2' position of the ribose sugar. The presence of this functional group causes the helix to adopt the A-form geometry rather than the B-form most commonly observed in DNA. This results in a very deep and narrow major groove and a shallow and wide minor groove. A second consequence of the presence of the 2'-hydroxyl group is that in conformationally flexible regions of an RNA molecule (that is, not involved in formation of a double helix), it can chemically attack the adjacent phosphodiester bond to cleave the backbone.

There are nearly 100 other naturally occurring modified nucleosides, of which pseudouridine and nucleosides with 2'-O-methylribose are the most common.

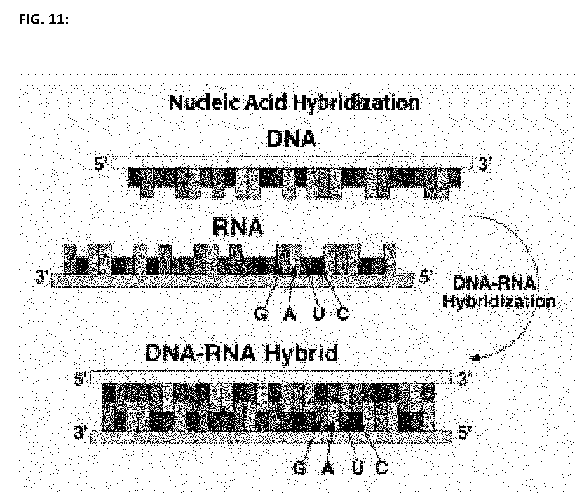

Herein, a "RNA/DNA" hybrid molecule is when an RNA strand hybridizes in reverse complementary manner with a DNA strand; see Fig. 11.

An antibody which is specific for such RNA/DNA hybrid molecule is also called an anti-RNA/DNA (hybrid) antibody. Once such antibody has bound to a RNA/DNA hybrid the resulting hybrid is called a RNA/DNA/antibody hybrid.

### DETAILED DESCRIPTION OF THE INVENTION

The herein described method differs from the previous methods in that the genomic regions of interest (target regions) are selectively enriched using unlabelled RNA probes. Such targeted enrichment is particular useful for a subsequent sequencing step because the target sequences only are subjected to analysis, thereby facilitating a significant reduction of DNA ballast by several orders of magnitude.

The herein described method is an enhancement of the SureSelect Target Enichment System described in the Example section but avoids the use of expensive labelled RNA probes (RNA baits). Further, the method of the invention extends applications of the DNA/RNA hybrid capture technology described in Digene patent US 6,228,578 B1 to genomic DNA of complex organisms, where there is a need for specifically enriching target sequences only, such as for the purpose of sequencing. Accordingly, the invention is suitable for selectively enriching and/or sequencing any DNA region of interest. These can be coding regions (exons) from any gene panel, e.g. metabolic or regulatory genes and oncogenes.

The digene patent WO 2007/056723 A2 discloses enrichment of a large number of viral target DNA sequences by hybridization to RNA probes followed by immunocapture of the DNA:RNA hybrid. However, it does not disclose sequencing of the DNA molecule as in the present invention and also does not comprise the adaptor nucleotides in its examples.

A similar method is disclosed in WO 2011/097528, comprising contacting a RNA sample with a DNA probe, such that DNA/RNA hybrids are formed from complementary strands, separating the hybrids from the sample and detecting the DNA probe in the hybrids, thereby indirectly detecting complementary RNA. The DNA probe comprises flanking signature sequences (primer binding sites) for amplification and bar code sequences for detection.

The method of WO 2011/097528 has several disadvantages in comparison to the present method. In the known method the RNA is indirectly detected via a DNA probe. The assay reliability in this case is lower in comparison to methods which determine directly the RNA. Further, the DNA probes are rather complex comprising a small sequence part complementary to the RNA to be detected and quite long flanking sequences. These probes are thus not only laborious to design but may also unintentionally bind to RNAs via the long flanking sequences, thereby generating false positive signals.

The present invention relates to a method for enriching and/or sequencing one or more target sequences of deoxyribonucleic acid (DNA) in a composition, comprising the steps of (a) providing a composition comprising one or more deoxyribonucleic acid (DNA) molecules, (b) hybridizing to said one or more DNA molecules one or more target specific ribonucleic acid (RNA) hybridization probes, thereby forming one or more RNA/DNA hybrids, (c) capturing the RNA/DNA hybrids with one or more antibodies being specific for such RNA/DNA hybrids, thereby forming one or more RNA/DNA/antibody hybrids, (d) isolating the one or more RNA/DNA/antibody hybrids, (e) amplifying the DNA molecules of the RNA/DNA/antibody hybrids if necessary, and (f) optionally, sequencing the DNA molecules of the RNA/DNA/antibody hybrids or the amplification product. The sequencing is preferably done by means of next generation sequencing.

In short, RNA probes being specific to one or more DNA molecules of interest (i.e. target specific RNA probes) present in the sample are hybridized to DNA (see Figure 11). It may be necessary to denature the DNA molecules to generate single-stranded DNA in order to efficiently hybridize the RNA probes to the DNA molecules. An anti-RNA/DNA hybrid antibody is provided that specifically binds to RNA/DNA hybrids thereby capturing said hybrids. The antibody including the RNA/DNA hybrid may then be isolated by suitable means, for example via Fc binding of free antibodies using protein A or by using antibodies bound to a solid surface. The method may optionally comprise washing the isolated RNA/DNA hybrids bound to the antibodies (RNA/DNA/antibody hybrids). The DNA molecules of the RNA/DNA/antibody hybrids may be then amplified and/or sequenced. The method is detailed in the following.

As outlined above, the target sequences are preferably selected from the group of coding regions (exons). It is further preferred that the coding regions are selected from the group of metabolic genes, regulatory genes and oncogenes.

Preferably the DNA molecules in the composition are a DNA fragment library for next generation sequencing and, optionally, the DNA fragments in said library comprise terminal universal adapter sequences.

A DNA fragment library may be created from whole DNA or genomic DNA. The DNA is isolated, fragmented and size selected. If necessary, 3' and/or 5' overhangs are repaired to generate blunt ends or fragments with an A-overhang preferably at the 3' end. At each end of a DNA fragment adapter sequences are ligated such that all DNA fragments within the library are flanked by the same sequence motif resulting in universal terminal adapter sequences. Preferably, a DNA fragment is flanked by two different universal terminal adapter sequences. The terminal adapter sequences can then be used to amplify the DNA fragment library.

Accordingly, it is preferred that the DNA molecules consist of a DNA fragment library, wherein (a) the DNA in the library has been fragmented and size selected followed, if necessary, by end repair in order to generate double stranded blunt end fragments or ends with an A-overhang, respectively, and wherein (b) the fragments have been ligated to double stranded adapter oligonucleotides in order to generate a fragment library with identical flanking sequences.

The present method makes use of target specific RNA probes. Current methods have the disadvantage that they involve labelled RNA probes, e.g. biotinylated RNA baits and/or make use of unspecific RNA probes. Labelled probes are expensive and cumbersome to produce. In contrast, there is no need for modifying or labelling the RNA probes used in the herein described method. As a consequence, the RNA probes are easy to produce and cost-effective. Hence, it is preferred that the RNA probes are unmodified and unlabelled. Unspecific RNA probes lead to the enrichment of unwanted DNA sequences, i.e. to an increased ballast for subsequent steps, such as a sequencing step.

In one aspect, the RNA probes may be synthesised RNA probes. In another aspect, the RNA probes may be isolated and purified from a biological sample. Preferably the RNA probes are synthesized first as DNA oligonucleotides containing a RNA polymerase promoter sequence at one end followed by in-vitro transcription (i.e. transcribed DNA probes).

The DNA/RNA hybrid capture technology is described in Digene patent US 6,228,578 B1. Herein, the anti-RNA/DNA hybrid antibodies are preferably selected from the group of monoclonal or polyclonal antibodies. It is particular preferred that the antibodies are monoclonal.

DNA/RNA specific antibodies are preferably coupled to a solid-phase for simple separation (e.g. magnetic beads) or may be in-solution and are separated by binding to a solid-phase coupled protein G which binds IgG antibodies. That is, the anti-RNA/DNA hybrid antibodies used in the herein described method are preferably bound to a solid surface. As will be understood by the skilled person in the art the orientation of the antibody is important for efficiently binding the RNA/DNA hybrid. The antibodies may be covalently coupled to the solid surface. The solid surface may be spherically shaped, for example round or elliptical. The diameter of a round or elliptical solid surface may be between 0.05 µm and 100 µm, preferably between 0.2 µm and 20 µm, more preferably between 1 µm and 10 µm. It is particularly preferred that the antibodies are bound to a particle preferably a magnetic particle.

If the antibodies are bound to a particle, the isolation step is preferably done by centrifugation or using a magnetic field, respectively.

The herein disclosed method may involve the step of amplifying the DNA molecules of the RNA/DNA/antibody hybrids depending on whether an amplification of the DNA molecules is necessary for the subsequent method step, e.g. analysis, quantification, detection and/or sequencing. For example, because the concentration of DNA molecules is too small.

Various amplification methods are known. In a preferred embodiment the amplification method is selected from the group of polymerase chain reaction (PCR), real-time PCR (rtPCR), helicase-dependent amplification (HDA) and recombinase-polymerase amplification (RPA).

The amplification method is either a non-isothermal method or an isothermal method. The non-isothermal amplification method may be selected from the group of polymerase chain reaction (PCR) (Saiki et al. (1985) Science 230:1350). The isothermal amplification method may be selected from the group of helicase-dependent amplification (HDA) (Vincent et al. (2004) EMBO rep 5(8):795-800), thermostable HDA (tHDA) (An et al. (2005) J Biol Chem 280(32):28952-28958), recombinase polymerase amplification (RPA) (Piepenburg et al. (2006) PloS Biol 4(7):1115-1120).

By "isothermal amplification reaction" in context of the present invention it is meant that the temperature does not significantly change during the reaction. In a preferred embodiment the temperature of the isothermal amplification reaction does not deviate by more than 10 °C, preferably by not more than 5 °C, even more preferably not more than 2 °C during the main enzymatic reaction step where amplification takes place.

Depending on the method of isothermal amplification of nucleic acids different enzymes are required for the amplification reaction. Known isothermal methods for amplification of nucleic acids are the above mentioned, wherein the at least one mesophilic enzyme for amplifying nucleic acids under isothermal conditions is selected from the group consisting of helicase, mesophilic polymerases, mesophilic polymerases having strand displacement activity, recombination proteins.

"Helicases" are known by those skilled in the art. They are proteins that move directionally along a nucleic acid phosphodiester backbone, separating two annealed nucleic acid strands (e.g. DNA, RNA, or RNA-DNA hybrid) using energy derived from hydrolysis of NTPs or dNTPs. Based on the presence of defined helicase motifs, it is possible to attribute a helicase activity to a given protein. The skilled artisan is able to select suited enzymes with helicase activity for the use in a method according to the present invention. In a preferred embodiment the helicase is selected from the group comprising helicases from different families: superfamily I helicases (e.g. dda, pcrA, F-plasmid tral protein helicase, uvrD), superfamily II helicases (e.g. recQ, NS3-helicase), superfamily III helicases (e.g. AAV rep Helicase), helicases from DnaB-like superfamily (e.g. T7 phage helicase) or helicases from Rho-like superfamily.

The amplification methods will comprise buffers, dNTPs or NTPs in addition to the enzymes required.

As used herein, the term "dNTP" refers to deoxyribonucleoside triphosphates. Non-limiting examples of such dNTPs are dATP, dGTP, dCTP, dTTP, dUTP, which may also be present in the form of labelled derivatives, for instance comprising a fluorescent label, a radioactive label, a biotin label. dNTPs with modified nucleotide bases are also encompassed, wherein the nucleotide bases are for example hypoxanthine, xanthine, 7-methylguanine, inosine, xanthinosine, 7-methylguanosine, 5,6-dihydrouracil, 5-methylcytosine, pseudouridine, dihydrouridine, 5-methylcytidine.

As used herein, the term "NTP" refers to ribonucleoside triphosphates. Non-limiting examples of such NTPs are ATP, GTP, CTP, TTP, UTP, which may also be present in the form of labelled derivatives, for instance comprising a fluorescent label, a radioactive label, a biotin label.

Preferably, the amplification method is the polymerase chain reaction (PCR) method.

A PCR reaction may consist of 10 to 100 "cycles" of denaturation and synthesis of a DNA molecule. In a preferred embodiment, the temperature at which denaturation is done in a thermocycling amplification reaction is between about 90 °C to greater than 95 °C, more preferably between 92 °C - 94 °C. Preferred thermocycling amplification methods include polymerase chain reactions involving from about 10 to about 100 cycles, more preferably from about 25 to about 50 cycles, and peak temperatures of from about 90 °C to greater than 95 °C, more preferably 92 °C - 94 °C. In a preferred embodiment, a PCR reaction is usually done using a DNA Polymerase originating from a thermophilic prokaryote to produce, in exponential quantities relative to the number of reaction steps involved, at least one target nucleic acid sequence, given (a) that the ends of the target sequence are known in sufficient detail that oligonucleotide primers can be synthesized which will hybridize to them and (b) that a small amount of the target sequence is available to initiate the chain reaction. Here the polymerase is preferably a polymerase with proofreading activity. The enzyme is preferably thermostable.

Primers for amplification may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981). One method for synthesizing oligonucleotides on a modified solid support is described in the US patent US4458006 A . It is also possible to use a primer which has been isolated from a biological source (such as a restriction endonuclease digest).

Preferred primers have a length of about 15 - 100, more preferably about 20 - 50, most preferably about 20 - 40 bases.

A further advantage of the present method is that the amplification step can be done without pre-isolating the DNA molecules from the RNA/DNA/antibody hybrid. Both the antibodies and the solid surface did not interfere with the amplification step. It is therefore not necessary to denature the hybrids in order to release the DNA molecules prior to amplifying the DNA. That is, the DNA molecules may be amplified directly on the isolated RNA/DNA/antibody hybrids.

The amplification step is preferably done with primers that bind the universal adapter sequences. Procedures for preparing primers are outlined above.

The present invention preferably involves the step of sequencing the one or more DNA molecules of the one or more RNA/DNA/antibody hybrids or, if desired or necessary, the amplification product. The current method has the advantage that it is not restricted to a particular sequencing method. However, a next generation sequencing method is preferred.

DNA sequencing techniques are of major importance in a wide variety of fields ranging from basic research to clinical diagnosis. The results available from such technologies can include information of varying degrees of specificity. For example, useful information can consist of determining whether a particular polynucleotide differs in sequence from a reference polynucleotide, confirming the presence of a particular polynucleotide sequence in a sample, determining partial sequence information such as the identity of one or more nucleotides within a polynucleotide, determining the identity and order of nucleotides within a polynucleotide, etc.

The sequencing step is preferably done by means of next generation sequencing. Manufacturers and technologies are Solexa/llumina which generate up to 600 Gigabase (Gb) of 36 or 150 bp, Roche/454 which generate up to 700 Mbp reads of 400-1000 bp, ABI/SOLiD™ which generate > 20 Gb/day reads of 35-75 bp, Helicos which generate 21 - 35 Gb reads of 25-45 bp and Complete Genomics (a service company). Other manufacturers include Pacific Bioscience commercializing PacBio RS.

The Solexa/llumina sequencing by synthesis technology is based on reversible dye-terminators. DNA molecules are first attached to primers on a slide and amplified so that local clonal colonies are formed (bridge amplification). Four types of reversible terminator bases (RT-bases) are added, and non-incorporated nucleotides are washed away. Unlike pyrosequencing, the DNA can only be extended one nucleotide at a time. A camera takes images of the fluorescently labelled nucleotides, then the dye along with the terminal 3' blocker is chemically removed from the DNA, allowing the next cycle (Brenner et al., Nature Biotechnol. 2000. 18(6):630-634).

The SOLiD™ ("Sequencing by Oligonucleotide Ligation and Detection") method (Life Technologies; WO 06/084132 A2) is based on the attachment of PCR amplified fragments of template nucleic acids via universal adapter sequences to magnetic beads and subsequent detection of the fragment sequences via ligation of labelled probes to primers hybridized to the adapter sequences. For the readout a set of four fluorescently labelled di-base probes are used. After read-out, parts of the probes are cleaved and new cycles of ligation, detection and cleavage are performed. Due to the use of di-base probes, two rounds of sequencing have to be performed for each template sequence.

PacBio RS is a single molecule real time sequencing (SMRT) platform based on the properties of zero-mode waveguides. A single DNA polymerase enzyme is affixed at the bottom of a ZMW with a single molecule of DNA as a template. The ZMW is a structure that creates an illuminated observation volume that is small enough to observe only a single nucleotide of DNA being incorporated by DNA polymerase. Each of the four DNA nucleotides is attached to one of four different fluorescent dyes. When a nucleotide is incorporated by the DNA polymerase, the fluorescent tag is cleaved off and diffuses out of the observation area of the ZMW where its fluorescence is no longer observable. A detector detects the fluorescent signal of the nucleotide incorporation, and the base call is made according to the corresponding fluorescence of the dye.

The current method has the advantage that it is not restricted to a particular sequencing method. If the sequencing step is done by next generation sequencing, it is preferred that the method applied is selected from the group of those described above.

The amplification product may additionally be detected and/or quantified prior to the sequencing step.

The detection step may be done by incorporating into the amplification product detectable probes, e.g. fluorescently labelled probes. A probe according to the present invention is an oligonucleotide, nucleic acid or a fragment thereof, which is substantially complementary to a specific nucleic acid sequence. Suitable hybridization probes include the LightCycler probe (Roche), the TaqMan probe (Life Technologies), a molecular beacon probe, a Scorpion primer, a Sunrise primer, a LUX primer and an Amplifluor primer.

The detection step may be alternatively done by using double-stranded DNA-binding dyes (e.g. SYBR Green) as reporters in a real-time PCR. A DNA-binding dye binds to all double-stranded DNA in PCR, causing fluorescence of the dye. An increase in DNA product during PCR therefore leads to an increase in fluorescence intensity and is measured at each cycle, thus allowing DNA concentrations to be quantified.

The quantification step may be based on quantitative real-time PCR using the techniques described before.

The present invention also relates to a kit comprising an antibody which is specific for a DNA/RNA hybrid molecule and adapter oligonucleotide, wherein optionally the antibody is bound to a magnetic particle, and additionally comprising one or more target specific RNA hybridization probes.

The constituents of the kit are the same as for the method disclosed above. For example, the RNA hybridization probes are preferably specific for target sequences selected from the group of coding regions (exons). The coding regions are preferably selected from the group of metabolic genes, regulatory genes and oncogenes. The RNA probes may be synthesised RNA probes. Alternatively, the RNA probes may be isolated and purified from a biological sample. Preferably the RNA probes are synthesized first as DNA oligonucleotides containing a RNA polymerase promoter sequence at one end followed by in-vitro transcription. For example, the Anti-RNA/DNA hybrid antibodies used herein are preferably bound to a solid surface. As will be understood by the skilled person in the art the orientation of the antibody is important for efficiently binding the RNA/DNA hybrid. The antibodies may be covalently coupled to the solid surface. The solid surface may be spherically shaped, for example round or elliptical. The diameter of a round or elliptical solid surface may be between 0.05 µm and 100 µm, preferably between 0.2 µm and 20 µm, more preferably between 1 µm and 10 µm. It is particularly preferred that the antibodies are bound to a magnetic particle.

### FIGURE CAPTIONS

**FIG. 1****:**
   Systematic overview of target enrichment technologies for next-generation sequencing.
**FIG. 2****:**

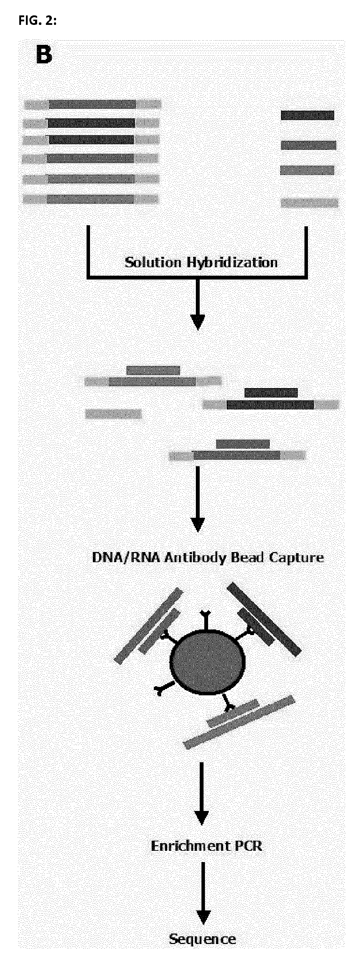

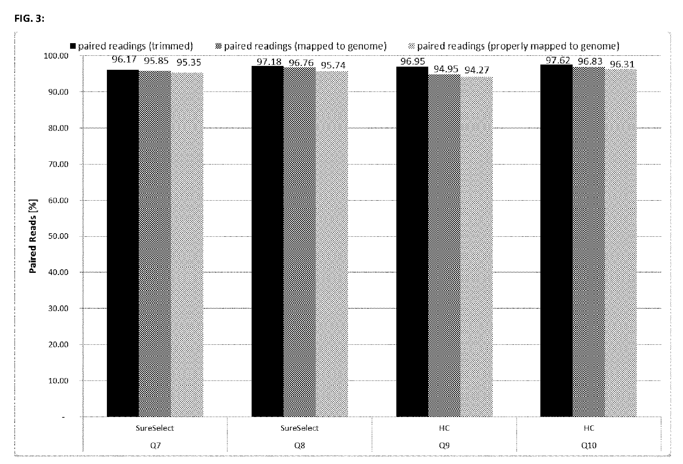

   Fig.2. Hybridization of single stranded adapter-ligated DNA fragments with RNA probes. DNA/RNA hybrid molecules bind to magnetic particles and are subsequently isolated by magnetic separation. Separated DNA fragments can be enriched by PCR prior sequencing. A. Hybridization of targeted DNA fragments with biotinylated RNA baits und purification with streptavidin coated magnetic beads. B. Hybridization of targeted DNA fragments with unlabelled and unmodified RNA probes and isolation of targeted hybrid molecules with antibody coated magnetic beads.
**FIG. 3****:**
   Percentage of sequence reads before and after mapping to the human genome (hg19) Percentages are normalized to the number of successful reads before quality assessment.
**FIG. 4****:**
   Description of region of interest (ROI) and region of design (ROD). ROI describes the targeted regions for enrichment (e.g. exon sequences E1-E5 including exon-intron boundaries). ROD describes the region which is covered by probes (a-e). Accordingly, ROD describes regions for which sequence data are expected. Gaps in regions of interest which could not be covered with suitable probes are labelled with f and g.
**FIG. 5****:**
   Sensitivities of the enrichment technologies. Percentage of ROI and ROD covered by at least one sequence. Percentages are related to the sizes of ROI and ROD, respectively.
**FIG. 6****:**
   Specificities of the enrichment technologies. Percentage of sequenced bases matching to ROD and ROI. Percentages are related to the number of sequenced bases which mapped to the human genome.
**FIG. 7****:**
   Percentage of ROD and ROI not covered by sequence data.
**FIG. 8****:**
   Boxplot for sequence coverage within ROI. The median value is between 2402 and 2867 for all 4 libraries investigated. The differences between upper (q3) and lower (q1) quartile are indicated in the lower lane.
**FIG. 9****:**
   Cumulative sequence coverage of ROI. All 4 curves have a similar shape. Approximately 93% of ROI are covered at least 1-fold (= sensitivity). At 100-fold coverage depending on the library between 87% and 90% (Q7: 90.47%, Q8: 88,13%, Q9: 88,33%, Q10: 86,97%) and at 1000-fold coverage at least 60% of ROI are covered by sequence data.
**FIG. 10****:**
   Normalized sequence coverage of ROI. It describes the evenness or sequence bias of the sequence coverage in ROI and provides important information for the experimental design in terms of expected sequence coverage. Example calculation for Q9: If at least 85% of the target region should be covered at least 30-fold (x-value = 0.1; y-value = 85%), the target region has to be covered in average more than 300-fold (x-value = 1 = average sequence coverage) or 65% of the target region should be covered at least 150-fold (x-value = 0.5). Furthermore the curves allow a comparison of sequence runs with varying number of readings as well as of different sample preparations. A high point of intersection with the y-axis and a smooth slope of the curve indicate an efficient sample preparation.
**FIG. 11****:**
   Fig. 11 shows a DNA/RNA hybrid structure.

### EXAMPLES

Next generation sequencing technologies allow generation of huge amounts of sequence information by massive parallel sequencing. However, most sequencing platforms do not yet have the capacity to sequence a complex genome like human in a single run cost effectively. On the other hand for many tasks it is rather necessary to sequence targeted regions of one or more samples.

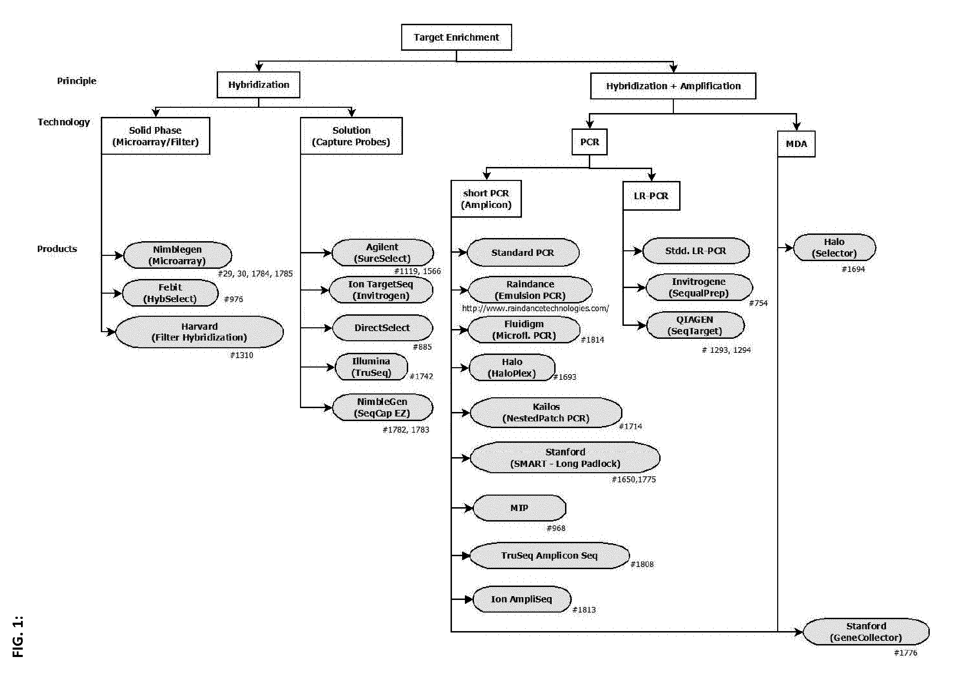

For this reason several target DNA enrichment protocols have been developed prior to next generation sequencing (Fig.1).

Whereas the so called "SureSelect" protocol requires RNA baits with affinity tag (i.e. biotin or hapten) on each bait sequence for hybridization and subsequent separation of a molecule or particle that binds to the affinity tag (e.g. magnetic beads coated with streptavidin, avidin or antibody that binds to the hapten or an antigen-binding fragment thereof), the herein disclosed method is based on in-solution hybridization of DNA library fragments to unmodified single stranded RNA probes without affinity tag followed by isolation of targeted DNA fragments by DNA/RNA specific antibodies. DNA/RNA specific antibodies are coupled to a solid-phase for simple separation (e.g. magnetic beads) or may be in-solution and are separated by binding to a solid-phase coupled G-protein specific secondary antibody.

The principle of the invention is shown in Fig. 2B

At first a fragment library is constructed. The DNA is fragmented and size selected followed by end repair to generate double stranded blunt end fragments or ends with "A" overhang, respectively. Such fragments are ligated to double stranded adapter oligonucleotides to generate a fragment library with identical flanking sequences. PCR allows arbitrary amplification of the library using primers matching to the adapter ends as well before as after targeted DNA enrichment.

For evaluation of the performance of this invention RNA probes were designed and synthesized for exon enrichment of 60 genes (Tab. 1) using the eArray Internet portal from Agilent (https://earray.chem.agilent.com/erray/).
In total 5942 RNA baits with 120 nucleotides each were synthesized, covering 91.83% of the targeted regions in the genome.

Biotinylated RNA baits were used for comparison of the targeted DNA enrichment using the "SureSelect" protocol as well as in the protocol of this invention based on antibody capturing. Biotinylation was necessary for binding to streptavidin beads used in the "SureSelect" protocol, but does not interfere with DNA/RNA antibodies or beads used in the invention.

The enrichment protocol of this invention includes following steps, (i) denaturation of the DNA fragment library, (ii) in-solution hybridization with RNA baits, (iii) binding of DNA/RNA hybrids to antibody coated magnetic beads, (iv) magnetic separation of targeted DNA fragments, (v) repeated wash steps to remove nonspecific attached DNAs, (vi) PCR for amplification of the enriched DNAs and introduction of sequencer specific linker sequences and optional barcoding of the library.
Denaturation of the DNA/RNA hybrids and removal of antibody coated beads is not necessary before PCR. Neither beads nor antibodies inhibit the PCR.
In the following sequencing results generated from 2 repeated DNA libraries after enrichment according to the "SureSelect" protocol (libraries Q7 and Q8) are compared with data obtained from 2 repeated enriched libraries according to the antibody based hybrid capture protocol of this invention (libraries Q9 and Q10). For sequencing the libraries were labeled with different index codes prior sequencing and loaded on one lane of a HiSeq 2000 sequencer from Illumina. Sequencing was carried out as paired end sequencing with 2x100 bp desired reading length. Sequences were analyzed using software package "Galaxy". Sequence data were mapped with program BWA to the human genome release GRCh37.p5 (hg19).

Table 3 summarizes the raw data of the 4 libraries generated with HiSeq 2000. For all 4 libraries similar amounts of raw data with comparable qualities were obtained (see average read length after trimming and average PHRED quality after trimming).

**Table 3**

| **Sample** | **Q7** | **Q8** | **Q9** | **Q10** |
|---|---|---|---|---|
| Method | Cancer60 SureSelect | Cancer60 SureSelect | Cancer60 HC | Cancer60 HC |
| | | | | |
| **# of RAW reads** | 20612924 | 22492202 | 18698880 | 19664166 |
| **# of RAW read pairs** | 10306462 | 11246101 | 9349440 | 9832083 |
| **# of trimmed reads (Q20)** | 20193992 | 22158970 | 18394757 | 19415982 |
| **# of read pairs after trimming** | 9911641 | 10928412 | 9064087 | 9598050 |
| **# of singletons after trimming** | 370710 | 302146 | 266583 | 219882 |
| **# of base pairs after trimming** | 1956095853 | 2172224180 | 1780044945 | 1896204827 |
| **average read length after trimming** | 96 | 98 | 96 | 97 |
| **average Phred quality after trimming** | 36.2 | 36.6 | 36 | 36.3 |

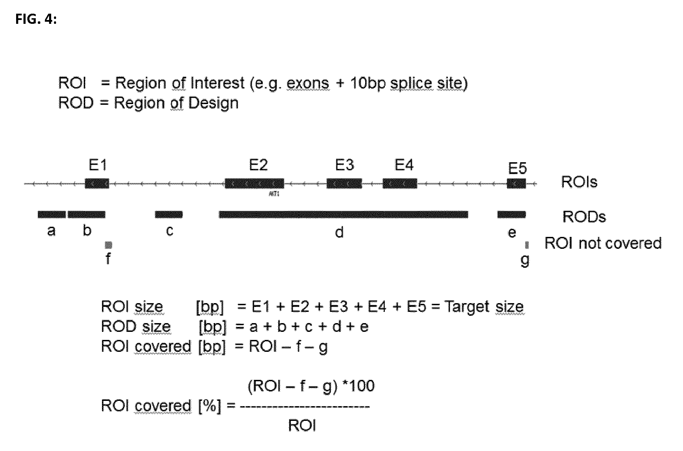

After quality trimming paired readings were mapped to the human genome (GRCh37.p5 (GCA_000001405.6) = hg19) und subsequently analyzed for their location within both the region of design (ROD) and region of interest (ROI) (Fig.4).

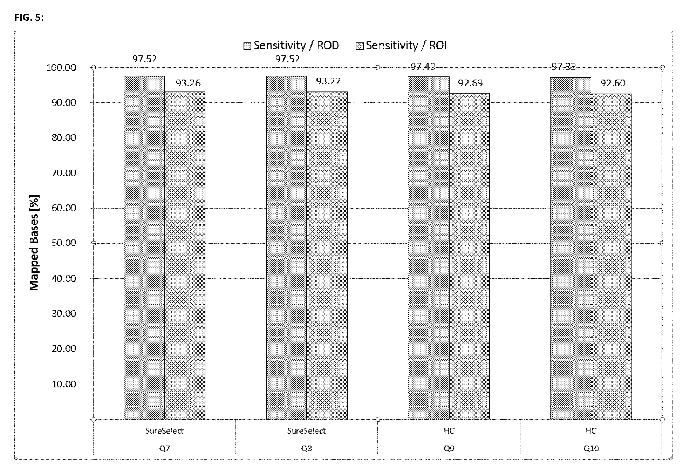

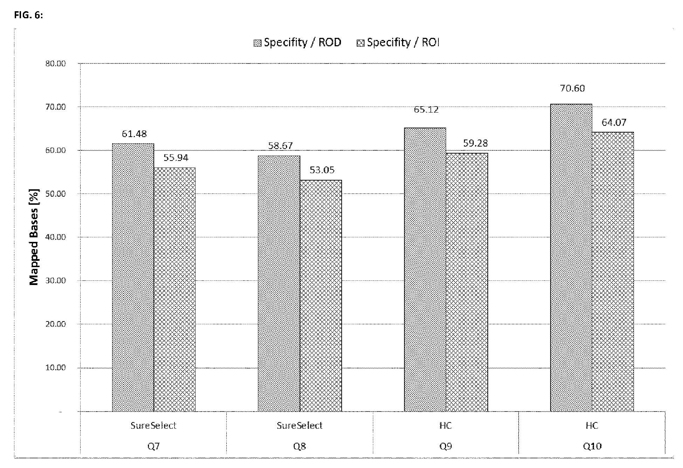

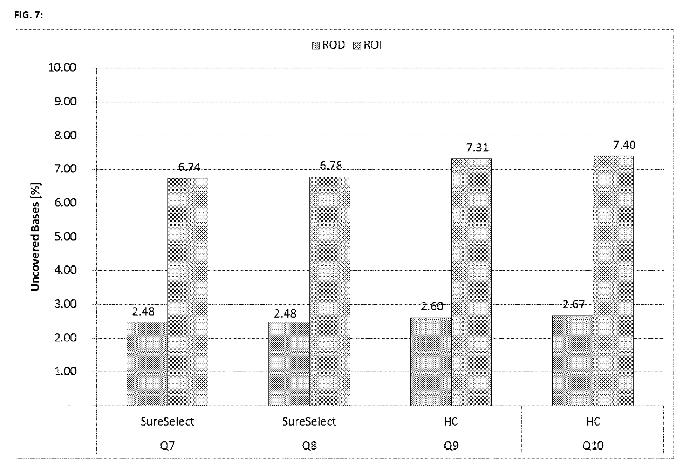

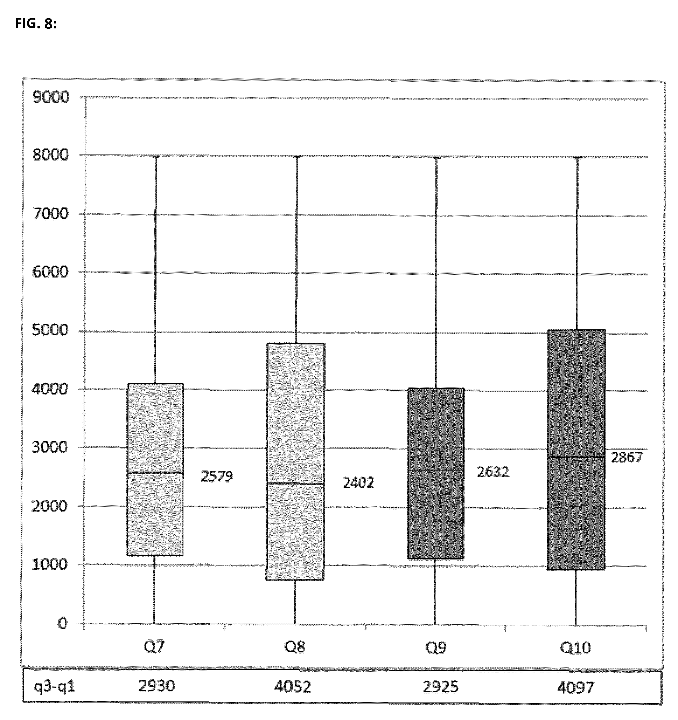

Following parameters were investigated: Sensitivity (How many nucleotides of targeted regions were covered with sequence data?) (Fig.5); Specificity (How many readings or nucleotides match to the targeted regions?) (Fig.6); Number and sizes of remaining gaps (Fig.7); Evenness of the sequence coverage (Fig.8)

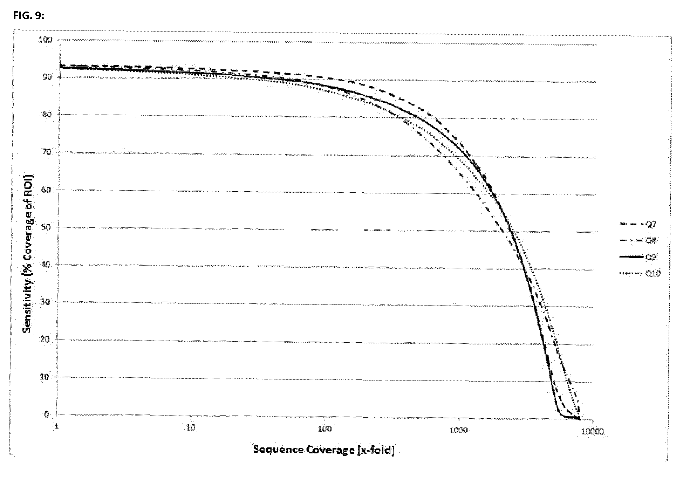

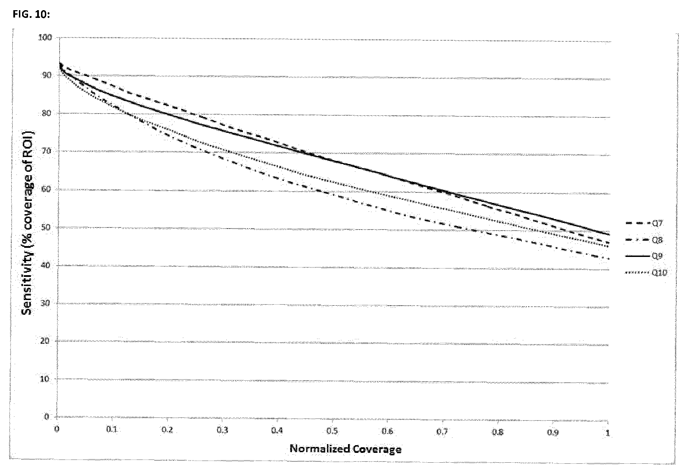

Plots in figures 9 and 10 summarize sensitivities and sequence coverage for all 4 libraries. From the data shown it was concluded that both enrichment technologies, SureSelect from Agilent and the hybrid capture technology of this invention, perform very similar in terms of sensitivity, specificity, number and size of gaps, and evenness of the sequence coverage. Consequently, the antibody based hybrid capture technology in this invention is a suitable alternative technology compared to biotin-streptavidin based RNA/DNA capturing, however, do not require producing expensive labelled RNA baits.

Tab.1. Target genes for exon enrichment. In total 1009 targeted regions were defined for probe design. The total size of the region of interest is 398908 bp.

**Table 1**

| | | | | | |
|---|---|---|---|---|---|
| ABL1 | CEBPA | FGFR3 | IDH2 | NRAS | RUNX1 |
| AKT1 | CRLF2 | FKBP9 | JAK2 | PDGFRA | SMAD4 |
| AKT3 | CSF1R | FLT3 | KIT | PIK3CA | SMARCB1 |
| ALK | CTNNB1 | FOXL2 | KRAS | PIK3R1 | SMO |
| APC | EGFR | GATA1 | MAP2K1 | PIK3R5 | STK11 |
| ATM | ERBB2 | GNAQ | MET | PTCH1 | TET2 |
| BRAF | EZH2 | GNAS | MPL | PTEN | TP53 |
| CBL | FBXW7 | HNF1A | NF2 | PTEN11 | TSHR |
| CDH1 | FGFR1 | HRAS | NOTCH1 | RB1 | VHL |
| CDKN2A | FGFR2 | IDH1 | NOTCH2 | RET | WT1 |

Tab.2. Overview of RNA probes. At first probes were designed with maximum 20 bases overlap to genomic repeat regions. For regions without suitable probes a second round of design with 40 bases which allowed an overlap to neighbouring repeat regions was performed. Thereafter probes were divided in probes with "normal" probes with up to 60% GC content and probes with increased GC content (>60%) and regions covered by a single probe (orphans). "Normal" probes cover the region of interest with 2-fold coverage and both "high" GC-content probes and orphans cover the region of interest 4-fold.

| Baits | 20bp repeat | 40bp repeat | bait tiling |
|---|---|---|---|
| | | | |
| normal | 2386 | 2641 | 2x |
| High GC | 369 | 300 | 4x |
| Orphans | 234 | 12 | 4x |
| | | | |
| total | 5942 | baits | |
| length | 120 | nucleotides | |

## Claims

1. Method for enriching one or more target sequences of a deoxyribonucleic acid (DNA) in a composition, comprising the steps of:
(a) providing a composition comprising one or more deoxyribonucleic acid (DNA) molecules,
(b) hybridizing to said one or more DNA molecules, one or more target specific ribonucleic acid (RNA) hybridization probes, thereby forming one or more RNA/DNA hybrids,
(c) capturing the RNA/DNA hybrids with one or more antibodies being specific for such RNA/DNA hybrids, thereby forming one or more RNA/DNA/antibody hybrids,
(d) isolating the one or more RNA/DNA/antibody hybrids,
(e) amplifying the one or more DNA molecules of the one or more RNA/DNA/antibody hybrids if necessary, and
(f) sequencing the DNA molecules of the RNA/DNA/antibody hybrids or the amplification product, wherein the sequencing is preferably done by means of next generation sequencing.

2. Method according to claim 1, wherein the target sequences are selected from the group of coding regions (exons).

3. Method according to claim 2, wherein the coding regions are selected from the group of metabolic genes, regulatory genes and oncogenes.

4. Method according to any of the previous claims, wherein the DNA molecules in the composition are a DNA fragment library for next generation sequencing and, optionally, the DNA fragments in said library comprise terminal universal adapter sequences.

5. Method according to any of the previous claims, wherein the DNA molecules consist of a DNA fragment library, wherein
(a) the DNA in the library has been fragmented and size selected followed, if necessary, by end repair in order to generate double stranded blunt end fragments or ends with an A-overhang, respectively, and wherein,
(b) the fragments have been ligated to double stranded or partially double stranded adapter oligonucleotides in order to generate a fragment library with identical flanking sequences.

6. Method according to any of the previous claims, wherein the RNA probes are unmodified and unlabelled.

7. Method according to any of the previous claims, wherein the RNA probes are synthesised RNA probes, transcribed DNA probes, or are isolated and purified from a biological sample.

8. Method according to any of the previous claims, wherein the antibodies are bound to a solid surface, preferably to a magnetic particle.

9. Method according to any of the previous claims, wherein, if the antibodies are bound to a magnetic particle, the isolation step is done with a magnetic field and optionally comprise washing the isolated RNA/DNA/antibody hybrids.

10. Method according to any of the previous claims, wherein the DNA molecules are amplified directly on the isolated RNA/DNA/antibody hybrids.

11. Method according to any of the previous claims, wherein the amplification step is done with primers that bind the universal adapter sequences.

12. Method according to any of the previous claims, wherein RNA is enzymatically digested prior to sequencing.

13. Kit suitable for the method of claim 1 comprising an antibody which is specific for a DNA/RNA hybrid molecule and terminal universal adapter oligonucleotides, wherein optionally the antibody is bound to a magnetic particle, and additionally comprising one or more target specific RNA hybridization probes.

14. Kit according to claim 13, wherein the RNA hybridization probes are specific for target sequences selected from the group of coding regions (exons).

15. Kit according to claim 14, wherein the coding regions are selected from the group of metabolic genes, regulatory genes and oncogenes.

## Patentansprüche

1. Methode zur Anreicherung einer oder mehrerer Zielsequenzen einer Desoxyribonukleinsäure (DNA) in einer Zusammensetzung, umfassend der Schritte:
(a) Bereitstellen einer Zusammensetzung umfassend einer oder mehrerer Desoxyribonukleinsäure- (DNA) Moleküle,
(b) Hybridisieren einer oder mehrerer Zielsequenz-spezifischer Ribonukleinsäure-(RNA) Hybridisierungssonden an besagte eine oder mehrere DNA-Moleküle, wobei eine oder mehrere RNA/DNA-Hybride gebildet werden,
(c) Einfangen der RNA/DNA-Hybride mit einem oder mehreren Antikörpern, die spezifisch für solch RNA/DNA-Hybride sind, wobei eine oder mehrere RNA/DNA/Antikörper-Hybride gebildet werden,
(d) Isolation von einer oder mehreren RNA/DNA/Antikörper-Hybriden,
(e) wenn nötig, Amplifikation einer oder mehrerer DNA-Moleküle, der einen oder mehreren RNA/DNA/Antikörper-Hybriden, und
(f) Sequenzierung der DNA-Moleküle der RNA/DNA/Antikörper-Hybride oder der Amplifikationsprodukte, wobei die Sequenzierung vorzugsweise mittels Next-Generation-Sequenzierung gemacht wird.

2. Methode nach Anspruch 1, wobei die Zielsequenzen ausgewählt sind aus der Gruppe von kodierenden Regionen (Exons).

3. Methode nach Anspruch 2, wobei die kodierenden Regionen ausgewählt sind aus der Gruppe von Stoffwechselgenen, regulatorischen Genen und Onkogenen.

4. Methode nach einem der vorherigen Ansprüche, wobei die DNA-Moleküle der Zusammensetzung eine DNA-Fragment-Bibliothek für Next-Generation-Sequenzierung sind und, optional, die DNA-Fragmente der besagten Bibliothek, terminale universelle Adaptersequenzen umfassen.

5. Methode nach einem der vorherigen Ansprüche, wobei die DNA-Moleküle aus einer DNA-Fragment-Bibliothek bestehen, wobei
(a) die DNA der Bibliothek fragmentiert wurde und die Größenselektion, falls nötig, durch Reparatur der Enden erfolgte, um doppelsträngige glattendige Fragmente oder Enden mit einem A-Überhang zu generieren, und wobei,
(b) die Fragmente an doppelsträngige oder teilweise doppelsträngige Adapter-Oligonukleotide ligiert wurden um eine Fragment-Bibliothek mit identischen flankierenden Sequenzen zu generieren.

6. Methode nach einem der vorherigen Ansprüche, wobei die RNA-Sonden unverändert und unmarkiert sind.

7. Methode nach einem der vorherigen Ansprüche, wobei die RNA-Sonden synthetisierte RNA-Sonden, transkribierte DNA-Sonden, oder aus einer biologischen Probe isoliert und gereinigt sind.

8. Methode nach einem der vorherigen Ansprüche, wobei die Antikörper an eine feste Oberfläche gebunden sind, vorzugsweise an magnetische Partikel.

9. Methode nach einem der vorherigen Ansprüche, wobei, wenn die Antikörper an magnetische Partikel gebunden sind, der Isolationsschritt mit einem magnetischen Feld erfolgt und optional, waschen der isolierten RNA/DNA/Antikörper-Hybride umfasst.

10. Methode nach einem der vorherigen Ansprüche, wobei die DNA-Moleküle direkt auf den isolierten RNA/DNA/Antikörper-Hybriden amplifiziert werden.

11. Methode nach einem der vorherigen Ansprüche, wobei der Amplifikationsschritt mit Primern gemacht wird, die an die universellen Adaptersequenzen binden.

12. Methode nach einem der vorherigen Ansprüche, wobei die RNA vor der Sequenzierung enzymatisch verdaut wird.

13. Kit, geeignet für die Methode aus Anspruch 1, umfassend eines Antikörpers der spezifisch für ein DNA/RNA-Hybridmolekül und terminale universelle Adapter-Oligonukleotide ist, wobei, optional, der Antikörper an magnetische Partikel gebunden ist, und zusätzlich eine oder mehrere Zielsequenz-spezifische RNA-Hybridisierungssonden umfasst.

14. Kit nach Anspruch 13, wobei die RNA-Hybridisierungssonden spezifisch für Zielsequenzen sind, ausgewählt aus der Gruppe von kodierenden Regionen (Exons).

15. Kit nach Anspruch 14, wobei die kodierenden Regionen ausgewählt sind aus der Gruppe von Stoffwechselgenen, regulatorischen Genen und Onkogenen.

## Revendications

1. Procédé pour enrichir une ou plusieurs séquences cibles d'un acide désoxyribonucléique (DNA) dans une composition, comprenant les étapes :
(a) apport d'une composition comprenant une ou plusieurs molécules d'acide désoxyribonucléique (ADN),
(b) hybridation à ladite une ou plusieurs molécules d'ADN d'une ou plusieurs sondes d'hybridation d'acide ribonucléique (ARN) spécifiques à la cible, formant ainsi un ou plusieurs hybrides ARN/ADN,
(c) capture des hybrides ARN/ADN avec un ou plusieurs anticorps spécifiques pour de tels hybrides ARN/ADN, formant ainsi un ou plusieurs hybrides ARN/ADN/anticorps,
(d) isolement de un ou plusieurs hybrides ARN/ADN/anticorps,
(e) amplification de la ou des molécules d'ADN du ou des hybrides ARN/ADN/anticorps si nécessaire, et
(f) séquençage des molécules d'ADN des hybrides ARN/ADN/anticorps ou du produit d'amplification, dans lequel le séquençage est de préférence effectué au moyen de séquençage de prochaine génération.

2. Procédé selon la revendication 1 dans lequel les séquences cibles sont choisies du groupe des régions codantes (exons).

3. Procédé selon la revendication 2 dans lequel les régions codantes son choisies du groupe des gènes métaboliques, des gènes régulateurs et des oncogènes.

4. Procédé selon l'une quelconque des revendications précédentes dans lequel les molécules d'ADN dans la composition sont une banque de fragments d'ADN pour le séquençage de prochaine génération et, facultativement, les fragments d'ADN de ladite banque comprennent des séquences terminales d'adaptateur universel.

5. Procédé selon l'une quelconque des revendications précédentes dans lequel les molécules d'ADN consistent en une banque de fragments d'ADN, où
(a) l'ADN de la banque a été fragmenté et sélectionné par taille suivi, si nécessaire, de réparation des extrémités afin de générer respectivement des fragments double brins avec des extrémités franches ou des extrémités A saillantes, et où
(b) les fragments ont été ligaturés à des oligonucléotides double brin ou partiellement double brin afin de générer une banque de fragments avec des séquences flanquantes identiques.

6. Procédé selon l'une quelconque des revendications précédentes dans lequel les sondes ARN ne sont pas modifiées et pas marquées.

7. Procédé selon l'une quelconque des revendications précédentes dans lequel les sondes ARN sont des sondes ARN synthétisées, des sondes ADN transcrites, ou sont isolées et purifiées d'un échantillon biologique.

8. Procédé selon l'une quelconque des revendications précédentes dans lequel les anticorps sont liés à une surface solide, de préférence à une particule magnétique.

9. Procédé selon l'une quelconque des revendications précédentes dans lequel si les anticorps sont liés à une particule magnétique, l'étape d'isolation est effectuée avec un champs magnétique et facultativement comprend laver les hybrides ARN/ADN/anticorps isolés.

10. Procédé selon l'une quelconque des revendications précédentes dans lequel les molécules d'ADN sont amplifiées directement sur les hybrides ARN/ADN/anticorps isolés.

11. Procédé selon l'une quelconque des revendications précédentes dans lequel l'étape d'amplification est effectuée avec des amorces qui se lient aux séquences d'adaptateur universel.

12. Procédé selon l'une quelconque des revendications précédentes dans lequel l'ARN est enzymatiquement digéré avant le séquençage.

13. Kit convenant pour un procédé selon la revendication 1 comprenant un anticorps qui est spécifique pour une molécule d'hybride ADN/ARN et des oligonucléotides adaptateurs universels terminaux, dans lequel l'anticorps est facultativement lié à une particule magnétique, et comprenant en outre une ou plusieurs sondes d'hybridation d'ARN spécifiques d'une cible.

14. Kit selon la revendication 13 dans lequel les sondes d'hybridation d'ARN sont spécifiques pour des séquences cibles choisies du groupe des régions codantes (exons).

15. Kit selon la revendication 14 dans lequel les régions codantes sont choisies du groupe des gènes métaboliques, des gènes régulateurs et des oncogènes.
